# EUROPÄISCHE PATENTSCHRIFT

(11) **EP 0 899 555 B1**
(45) Veröffentlichungstag und Bekanntmachung des Hinweises auf die Patenterteilung: **27.02.2002**
(21) Anmeldenummer: 98890250.8
(22) Anmeldetag: 25.08.1998
(51) Int. Cl.: G01N 3/08, G01N 3/04, G01N 33/36

(54) **Verfahren zur Bestimmung der Festigkeitseigenschaften von langgestrecktem, textilem Prüfgut und Vorrichtung zur Durchführung des Verfahrens**
Method and device dor determining the strength of elongated textile specimens
Méthode et dispositif de détermination de la résistance d'échantillons textiles allongés

(30) Priorität: 02.09.1997 CH 205197
(43) Veröffentlichungstag der Anmeldung: 03.03.1999
(73) Patentinhaber: LENZING AKTIENGESELLSCHAFT, 4860 Lenzing (AT)
(72) Erfinder: Roos, Gerold, 8610 Uster (CH)
(74) Vertreter: Köhler-Pavlik, Johann, Dipl.-Ing.

(56) Entgegenhaltungen:
- EP-A- 0 429 376
- DE-A- 4 329 051
- DE-C- 3 942 111
- US-A- 4 148 218

## Beschreibung

Die vorliegende Erfindung betrifft ein Verfahren zur Bestimmung der Festigkeitseigenschaften von langgestrecktem, textilem Prüfmaterial, bei weichem das Prüfmaterial zwischen einer feststehenden und einer verschiebbaren Klemmvorrichtung eingespannt und gedehnt wird und das Prüfmaterial zwischen den Klemmvorrichtungen durch mindestens eine frei laufende Messrolle um einen Winkel umgelenkt wird.

Ein Verfahren der gegenständlichen Art ist beispielsweise in dem Artikel "Meßtechnische Untersuchungen über die Eignung eines neuen Schnellverfahrens zur Ermittlung der Reißkraft von fortlaufend bewegten Fäden bzw. Gespinsten und Zwirnen" von Obering. Herbert Stein und Dipl.-Phys. Siegfried Hobe, Westdeutscher Verlag Köln und Opladen 1966, bekannt.

Bekannte Zugprüfgeräte für die Prüfung von Garnen, Zwirnen schmalen Bändern und anderen flexiblen Materialien sind mit zwei Klemmvorrichtungen ausgerüstet. Diese sind vertikal übereinander in einem wählbaren Abstand angebracht. Die obere Klemmvorrichtung ist über eine starre Verbindung mit einer Kraftmesszelle verbunden. Die untere Klemmvorrichtung ist über einen Antrieb vertikal verschiebbar. Ein in den Klemmvorrichtungen eingespannter Prüfling wird durch die Abwärtsbewegung der unteren Klemmvorrichtung einer Zugbelastung ausgesetzt. Die auftretende Zugkraft im Prüfling wird durch die obere Klemmvorrichtung auf die Kraftmesszelle übertragen. Der von der unteren Klemme vom Prüfbeginn bis zum Bruch des Prüflings zurückgelegte Weg wird über einen mit dem Antrieb verbundenen Inkrementgeber als Dehnung des Prüflings registriert.

Diese Messanordnung ist mit Mängeln behaftet. Die Koppelung von Klemmvorrichtung und Kraftmesszelle stellt für die Kraftmesszelle eine, die Empfindlichkeit der Kraftmesszelle beeinträchtigende Vorlast dar. Diese Vorlast erhöht die Massenträgheit und senkt die Eigenfrequenz des Kraftmesssystems. Besonders störend ist dieser Einfluss, wenn die auftretenden Reißkräfte im Verhältnis zur maximal möglichen Belastung der Kraftmesszelle klein sind. Die Prüfgeräte selbst sind für Zugkräfte von 500N bis 5KN und mehr ausgelegt. Man begegnet diesem Problem mit austauschbaren Messzellen und Klemmvorrichtungen. Für ein umfangreiches Prüfmaterialsortiment können die zu prüfenden Reißkräfte von wenigen CN bis in den Bereich von einigen KN reichen. Die Anschaffung der dafür erforderlichen Kraftmesszellen und Klemmvorrichtungen ist mit hohen Kosten verbunden.

Ein weiterer Mangel in der Messanordnung dieser Geräte besteht darin, dass für die Dehnungsmessung die Abstandsänderung der Klemmvorrichtungen herangezogen wird. Während einer Zugprüfung wird das Prüfmaterial in den Klemmvorrichtungen eingespannt. Die im Prüfmaterial herrschende Zugspannung setzt sich dabei in Zugrichtung in beiden Klemmvorrichtungen fort und erreicht erst innerhalb der Klemmflächen den Wert Null. Dieser Zugkraftabbau innerhalb der Klemmflächen hat eine Dehnung zur Folge, die mit der beschriebenen Messmethode nicht erfasst wird. Der gemessene Dehnungswert ist zu groß.

Die effektive Dehnung ist die Veränderung des Klemmenabstandes minus zweimal den Wert des "Klemmenschlupfes". Bei Prüfmaterialien mit kleinen Dehnungswerten ist dieser Messfehler zu vernachlässigen, im besonderen wenn dieser auf eine große Einspannlänge bezogen wird. Bei elastischen Prüfmaterialien mit hoher Dehnung und Querkontraktion, wie gummiähnliche Materialien oder teilverstreckten Chemiegarnen aber auch bei weichgeglühten Metalldrähten nimmt dieser Fehler eine nicht mehr zu vernachlässigende Größe an. Dieser Messfehler vergrößert sich noch beträchtlich, wenn die Einspannlänge vom Standardwert 500 mm auf 100 mm oder gar 50 mm verringert wird. Verkürzte Einspannlängen werden gewählt, um elastische Prüfmaterialien innerhalb des größtmöglichen Klemmabstandes bis zum Bruch zu belasten.

Die DE 39 42 111 C1 beschreibt einen Standardreißkraftprüfer, der die zur Klemmung benötigten Kräfte durch Umschlingungen reduziert, wodurch weniger Klemmenschlupf auftritt.

Die US 4 148 218 A beschreibt eine Vorrichtung zur dynamischen Spannungsprüfung, bei der anstelle von Klemmen ein Riemen vorgesehen ist. Mit Hilfe der Seilreibung und der Andrückkraft des Riemens wird ein Faden gespannt und durch Bewegung des Riemens dieser gedehnt. Dabei handelt es sich nicht um einen Reißkraftprüfer der gegenständlichen Art, bei dem auch nicht der Klemmenschlupf gemessen wird.

Durch die vorliegende Erfindung soll der Einfluss des Gewichts der Klemmvorrichtung auf das Kraftmesssystem beseitigt und durch Erfassen des Klemmenschlupfes die Genauigkeit der Dehnungsmessung erhöht werden.

Diese Aufgabe wird erfindungsgemäß dadurch gelöst, dass die Gesamtdehnung des Prüfmaterials einschließlich des Klemmenschlupfes des Prüfmaterials und die Dehnung des Prüfmaterials zumindest in einem der Bereiche zwischen Klemmvorrichtung und benachbarter Messrolle einschließlich des Klemmenschlupfes in dehnungsproportionale Signale umgewandelt wird, und dass diese Signale ausgewertet werden.

Eine bevorzugte Ausführungsform des erfindungsgemäßen Verfahrens ist dadurch gekennzeichnet, dass durch die Umlenkung des Prüfmaterials um einen Winkel ein Kräftepaar gebildet wird, dessen resultierende Kraft zur Bestimmung der Festigkeitseigenschaften herangezogen wird.

Die Erfindung betrifft weiter eine Vorrichtung zur Durchführung des genannten Verfahrens, mit einer feststehenden und einer verschiebbaren Klemmvorrichtung, zwischen denen eine Messstrecke gebildet ist, innerhalb welcher mindestens eine freilaufende Messrolle zur Umlenkung des Prüfmaterials um den genannten Winkel angeordnet ist. Die erfindungsgemäße Vorrichtung ist dadurch gekennzeichnet, dass mindestens eine der Messrollen mit einem Inkrementgeber ausgerüstet ist, durch welchen eine Umwandlung der auf die Messrollen als Drehbewegung übertragene Dehnung des Prüfmaterials in den Bereichen zwischen Klemmvorrichtung und benachbarter Messrolle einschließlich des aus den Klemmvorrichtungen herauskriechenden Klemmenschlupfes in dehnungsproportionale Signale erfolgt, dass weiters ein die Veränderung des Abstandes der Klemmvorrichtungen überwachender Inkrementgeber vorgesehen ist, und dass die von diesem erfassten Dehnungswerte in dehnungsproportionale Signale umgewandelt und zusammen mit den dehnungsproportionalen Signalen des zumindest einen genannten Inkrementgebers einem Auswertegerät zugeführt sind. Während eines Prüfvorganges wird das Prüfmaterial zwischen den Klemmen durch eine konstante Vergrößerung des Klemmenabstandes gedehnt. Als Dehnungsgrösse wird die Veränderung des Abstandes der Klemmvorrichtung zwischen dem Beginn der Prüfung und dem Bruch des Prüfmaterials gemessen. Diese Abstandsänderung wird von einem, mit dem Antrieb für die bewegliche Klemmvorrichtung verbundenen Inkrementgeber erfasst und als dehnungsproportionaler Wert zum Auswertegerät weitergeleitet. Die Dehnungsanteile der Materialstücke, die ausserhalb der vertikalen Prüfstrecke anfallen, einschließlich des Klemmenschlupfes, setzen die Messrollen in Drehung. Die Drehwinkel der beiden Messrollen werden durch die in den Messrollen eingebauten Inkrementgeber ebenfalls in dehnungsproportionale Werte umgewandelt und dem Auswertegerät übermittelt.

Eine bevorzugte Ausführungsform der erfindungsgemäßen Vorrichtung ist dadurch gekennzeichnet, dass zwei Messrollen vorgesehen sind, von denen mindestens eine mit einer Kraftemesszelle in Verbindung steht.

Erfindungsgemäß ist die Klemmvorrichtung von der Kraftmesszelle getrennt. Zur Krafteinleitung ist auf der Kraftmesszelle eine Messrolle mit eingebautem Inkrementgeber angebracht. Die Messrolle der Kraftmesszelle und eine zweite Messrolle sind innerhalb der Prüfstrecke so angebracht, dass das Prüfmaterial auf den beiden Messrollen umgelenkt wird. Die Prüfstrecke mit dem Prüfmaterial zwischen den Klemmvorrichtungen hat dadurch einen trapezförmigen Verlauf.

Die Kraftmesszelle mit Messrolle ist dabei unterhalb der oberen Klemmvorrichtung montiert. Die zweite Messrolle, die ebenfalls mit einem Inkrementgeber bestückt ist, ist in der Nähe der unteren, verschiebbaren Klemmvorrichtung angebracht. Während des Prüfvorganges ist das Prüfmaterial in den Klemmvorrichtungen eingespannt und über die beiden Messrollen umgelenkt. Dabei kommt dem Auflagewinkel des Prüfmaterials auf der mit der Kraftmesszelle verbundenen Messrolle eine besondere Bedeutung zu. Das auf dieser Messrolle anfliegende, unter Zugkraft stehende Prüfmaterial bildet ein Kräftepaar. Der durch das Kräftepaar gebildete Winkel bestimmt die Größe der resultierenden Kraft, die über die Messrolle in die Kraftmesszelle eingeleitet wird. Durch die Wahl des Auflagewinkels zwischen 0° und 60° können Messzellen eingesetzt werden deren Maximalbelastung bedeutend niedriger ist als die zu prüfende Zugkraft. Wird als Auflagewinkel 60° (eingeschlossener Winkel 120°) gewählt, so entspricht die resultierende Kraft der Zugkraft im Prüfmaterial. Als Einspannlänge, auf welche die Dehnungswerte bezogen werden, kann bei der beschriebenen Messanordnung sowohl die Materiallänge zwischen den Klemmen als auch der Abstand zwischen den Messrollen definiert werden. Als Messlänge ist immer die Materiallänge zwischen den Klemmvorrichtungen mit Umlenkung über die beiden Messrollen definiert.

Ausgehend von den, von den Inkrementgebern übermittelten Dehnungswerten und deren zeitlichen Verlauf, wird im Auswertegerät die auf die gewählte Bezugslänge bezogene Dehnung berechnet. Unter Einbezug der während der Prüfung über die Kraftmesszelle gemessenen Zugkräfte und deren zeitliche Veränderung können weitere Prüfparameter und Kenngrößen des gemessenen Prüfmaterials bestimmt werden.

Nachfolgend wird die Erfindung anhand eines Ausführungsbeispiels und Zeichnungen näher erläutert. Es zeigen:
- Fig. 1: eine Ansicht eines Reißkraftgerätes in vereinfachter, schematischer Darstellung.
- Fig. 2: einen Ausschnitt aus Fig. 1 bei kleinem Abstand der Klemmvorrichtungen.
- Fig. 3: einen Schnitt durch den Klemmspalt einer Klemmvorrichtung mit dem Entstehen und dem Verlauf des Klemmenschlupfes. 3a bei Beginn, 3b während eines Prüfvorganges
- Fig. 4: das durch das Prüfmaterial auf der oberen Messrolle gebildete Kräftepaar mit der resultierenden Kraft.
- Fig. 5: eine Ansicht der oberen Messrolle mit Kraftmesszelle und Messrollen-Abdeckung
- Fig. 6: einen Schnitt durch eine der beiden Messrollen mit Abdeckung und Inkrementgeber

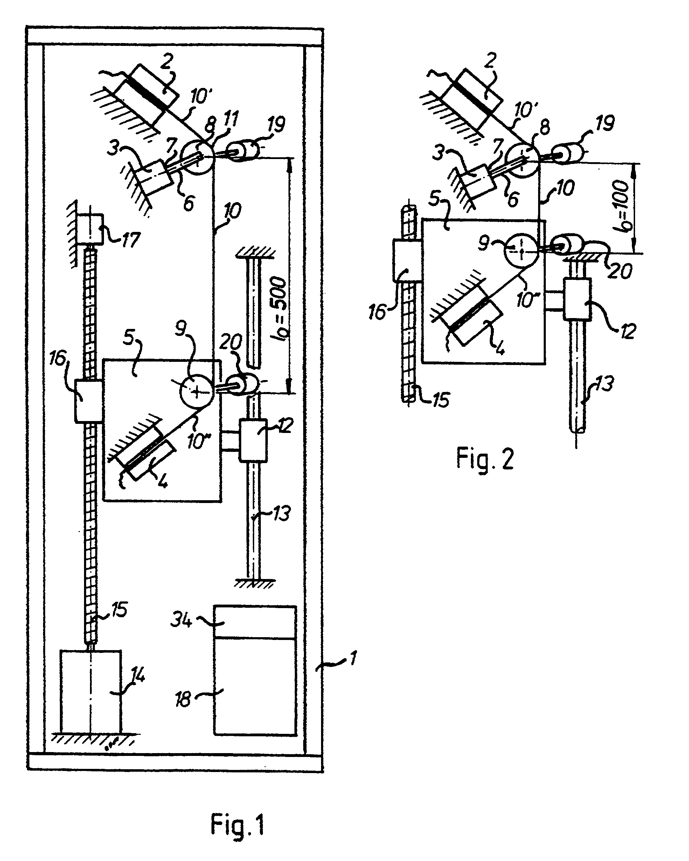

In Fig. 1 ist die Ansicht eines Reissprüfgerätes in vereinfachter Form dargestellt. Auf der Frontseite des schrankförmigen Gehäuses 1 sind die für die Belastung und die Erfassung der Reisskraft und Dehnung des Prüfmaterials erforderlichen Baugruppen angeordnet. Funktionsgruppen, die für die Erfindung nicht von Bedeutung sind werden weggelassen.

Die feststehende obere Klemmvorrichtung 2 und die Kraftmesszelle 3 sind unabhängig voneinander im oberen Teil der Gerätefront angebracht. Die verschiebbare Klemmvorrichtung 4 ist auf einem Schlitten 5 befestigt. Zur Einleitung der Zugkraft in die Kraftmesszelle 3 ist auf einer Lasche 6, die an der Krafteinleitungsfläche 7 der Kraftmesszelle 3 befestigt ist, eine drehbare leicht laufende Messrolle 8 angebracht, eine weitere Messrolle 9 ist auf einem beweglichen Schlitten 5 angebracht. Die Klemmvorrichtungen 2 und 4 und Messrollen 8 und 9 sind so angebracht und ausgerichtet, dass das Prüfmaterial 10 in einem trapezförmigen Verlauf durch die Prüfstrecke geführt wird. Unter Zugbelastung stehendes Prüfmaterial bildet an der Auflagestelle 11 auf der Messrolle 8 ein Kräftepaar P; P' von gleicher Grösse. Die resultierende Kraft R wird über die Lasche 6 in die Kraftmesszelle 3 eingeleitet. Im allgemeinen wird der die Resultierende R bestimmende Winkel a des Kräftepaares so gewählt, dass die auf die Kraftmesszelle 3 wirkende Kraftkomponente R 10 % bis 100 % der Zugkraft P beträgt.

Der die untere Klemmvorrichtung 4 und die zweite Messrolle 9 tragende Schlitten 5 ist durch ein Gleitstück 12 auf der Schiene 13 geführt. Durch den Motor 14, die mit dem Motor 14 verbundene Gewindespindel 15 und die am Schlitten 5 befestigte Mutter 16 kann der Schlitten 5 in vertikaler Richtung verschoben werden. Auf dem vom Motor 14 abgewandten Ende der Gewindespindel 15 ist ein Inkrementgeber 17 montiert, durch den das Anfahren der Einspannlänge l₀ über die Gerätesteuerung 18 gesteuert wird. Durch den Inkrementgeber 17 wird während einer Prüfung die als Dehnung interpretierte Vergrösserung des Klemmabstandes vom Beginn einer Prüfung bis zum Bruch des Prüflings in Form von wegproportionalen Impulsen erfasst. Der so erfasste Weg entspricht der Gesamtdehnung des Prüfmaterials einschliesslich dem aus den Klemmvorrichtungen 2, 4 kriechende Klemmenschlupf.

Die Dehnungsanteile der Prüfmaterialabschnitte 10' und 10" bewirken eine Drehbewegung der Messrollen 8 und 9. Mit den Messrollen 8 und 9 verbundene Inkrementgeber 19 und 20 erfassen die Drehbewertung der Messrollen 8 und 9 und wandeln diese in dehnungsproportionale Impulse um. Die Dehnungswerte der Inkrementgeber 17, 19 und 20 werden sowohl in der Grösse als auch in ihrem zeitlichen Verlauf im Auswertegerät 34 gespeichert. Nach dem Bruch des Prüfmaterials 10 beziehungsweise nach Abschluss einer Prüfserie werden mittels eines Auswerteprogramms die Dehnungswerte ermittelt.

Fig. 2 zeigt einen Ausschnitt aus Fig. 1 mit stark verkürzter Einspannlänge l₀=100 mm. Daraus ist ersichtlich, dass die Prüfmaterialabschnitte 10' und 10" und die Erfassung der Prüfkraft gleichgeblieben sind. Durch die Erfassung des aus den Klemmvorrichtungen 2 und 4 kriechenden Klemmenschlupfes wird die Genauigkeit der Dehnungsmessung bei kurzen Einspannlängen wesentlich verbessert.

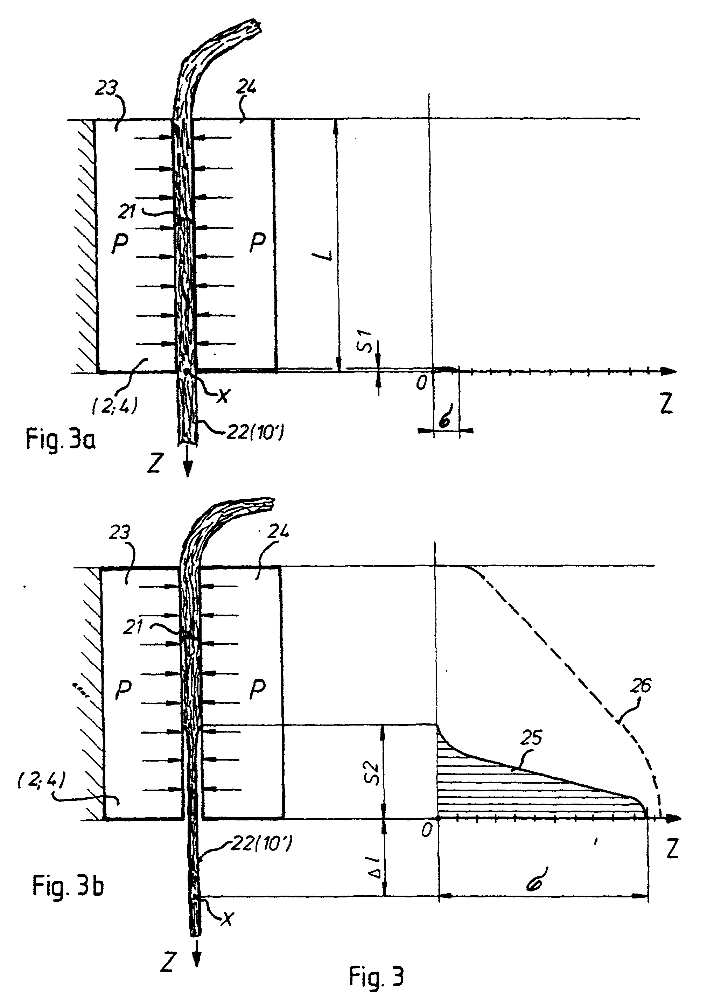

Fig. 3a und 3b zeigen Schnittdarstellungen durch den Klemmspalt 21 einer Klemmvorrichtung 2, 4 mit dem Entstehen und dem Verlauf des Klemmenschlupfes.

Fig. 3a zeigt den Klemmspalt 21 beim Beginn eines Prüfganges. Das Prüfmaterial 22 liegt zwischen der feststehenden 23 und der beweglichen Klemmbacke 24 . Die Klemmkraft P verteilt sich gleichmässig auf die Klemmlänge L. Mit Z ist die im Prüfmaterial 22 herrschende Zugkraft bezeichnet. X ist eine Markierung auf dem Prüfmaterial 22 kurz nach dem Belastungsbeginn. Auf nebenstehendem Diagramm ist die Spannung σ infolge der Zugkraft Z und des Klemmschlupfs S₁, aufgetragen.

Fig. 3b zeigt den Klemmspalt 21 kurz vor dem Bruch des eingespannten Prüfmaterials 22. Durch die Zugkraft Z und die dadurch verursachte Dehnung Δ1 hat sich das Prüfmaterial 22 eingeschnürt. Die Zugkraft Z hat sich auf der Strecke S₂ vom maximalen Wert Z auf Null abgebaut. Dieser Kraftabbau innerhalb der Klemmstrecke hat einen Klemmenschlupf Δ1 zur Folge. Die zuvor am Austritt aus dem Klemmspalt 21 angebrachte Markierung X hat sich um den Betrag Δ1 von den Klemmbacken 23, 24 entfernt. Im nebenstehenden Diagramm ist der Spannungsverlauf 25 innerhalb des Klemmspalts 21 dargestellt. Die punktierte Diagrammlinie 26 stellt den Fall des Durchrutschens des Prüfmaterials 22 durch den Klemmspalt 21 dar. Die Zugkraft Z beziehungsweise die Spannung σ hat erst hinter dem Klemmspalt 21 den Wert Z=0, σ=0 erreicht.

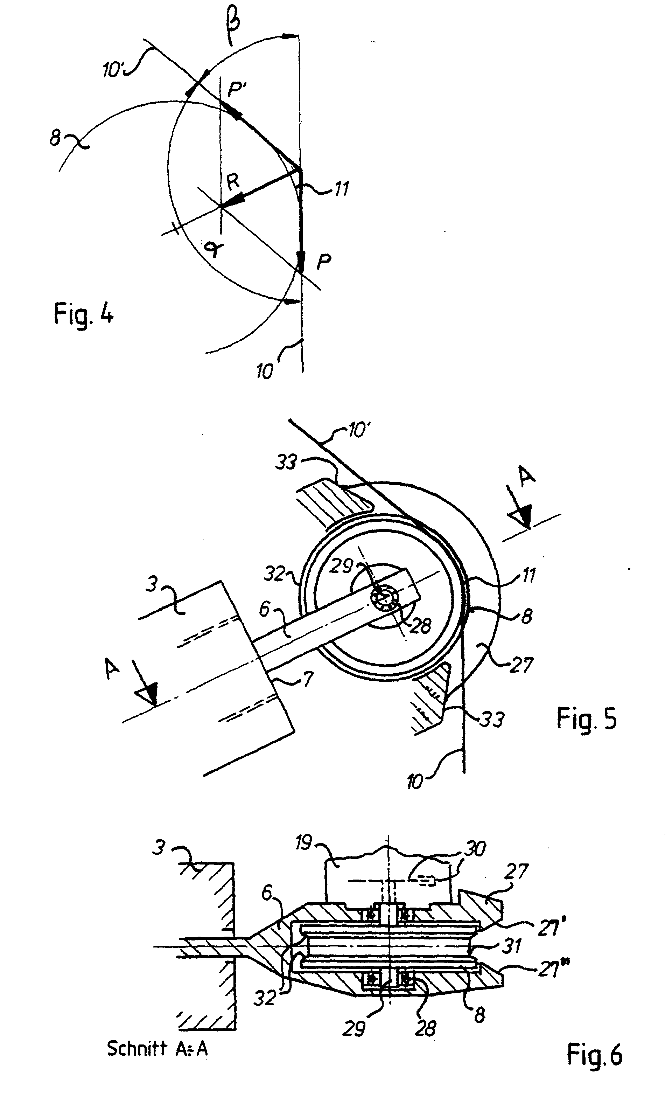

Fig. 4 zeigt das auf der oberen Messrolle 8 gebildete Kräftepaar P, P' mit der resultierenden, zur Messung der Zugkraft Z verwendeten Kraftkomponente R. Die Prüfmaterialabschnitte 10 und 10' liegen auf der oberen Messrolle 8 auf und bilden den eingeschlossenen Winkel α. P und P' sind die im Prüfmaterial 10 herrschenden Zugkräfte und R die resultierende Kraftkomponente die unter Beibehaltung der Vektorrichtung in die Kraftmesszelle 3 eingeleitet wird. Der Winkel β entspricht dem Umlenkwinkel.

Fig. 5 zeigt eine Ansicht der oberen Messrolle 8 mit Kraftmesszelle 3 und Abdeckung 27. Die über ein kugellager 28 gelagerte Messrolle 8 ist über die Lasche 6 mit der Krafteinleitungsfläche 7 der Kraftmesszelle 3 verbunden und zum Schutze vor eindringenden Materialresten und Staub durch die Abdeckung 27 abgedeckt. Die Einlaufschrägen 33 sind so gestaltet, dass das zu messende Prüfmaterial 10 ausschliesslich durch die Messrolle 8 geführt wird, und dass nach erfolgtem Bruch des Prüfmaterials 10 das zurückschlagende Materialende die Messrolle 8 nicht beschädigen kann.

Fig. 6 zeigt einen Schnitt längs der Linie A - A von Fig. 5. Auf der in Kugellagern 28 gelagerten Welle 29 ist ein Inkrementgeber 19 bestehend aus Inkrementscheibe und Abtasteinheit 30 angebracht. Die Lauffläche 31 der Messrolle 8 ist beidseitig mit angeschrägten Borden 32 versehen durch die ein Abgleiten des Prüfmaterials während des Einlegens in die Prüfstrecke verhindert wird. Die beiden angeschrägten Abdeckungsteile 27', 27" bilden eine berührungsfreie Labyrinthdichtung zwischen Messrolle 8 und Abdeckung 27 und verhindert das Eindringen von Staub und Materialresten.

## Patentansprüche

1. verfahren zur Bestimmung der Festigkeitseigenschaften von langgestrecktem, textilem Prüfmaterial (10), bei welchem das Prüfmaterial (10) zwischen einer feststehenden und einer verschiebbaren Klemmvorrichtung (2 bzw. 4) eingespannt und gedehnt wird und das Prüfmaterial (10) zwischen den Klemmvorrichtungen (2, 4) durch mindestens eine frei laufende Messrolle (8, 9) um einen Winkel (β) umgelenkt wird, **dadurch gekennzeichnet, dass** die Gesamtdehnung des Prüfmaterials (10) einschliesslich des Klemmenschlupfes (Δ1) des Prüfmaterials (10) und die Dehnung des Prüfmaterials (10) zumindest in einem der Bereiche zwischen Klemmvorrichtung (2, 4) und benachbarter Messrolle (8, 9) einschließlich des Klemmenschlupfes (Δ1) in dehnungsproportionale Signale umgewandelt wird, und dass diese Signale ausgewertet werden.

2. Verfahren nach Anspruch 1, **dadurch gekennzeichnet, dass** durch die Umlenkung des Prüfmaterials (10) um einen Winkel (β) ein Kräftepaar (P, P') gebildet wird, dessen resultierende Kraft (R) zur Bestimmung der Festigkeitseigenschaften herangezogen wird.

3. Vorrichtung zur Durchführung des Verfahrens nach Anspruch 1 oder 2, mit einer feststehenden und einer verschiebbaren Klemmvorrichtung (2 bzw. 4), zwischen denen eine Messstrecke (lₒ) gebildet ist, innerhalb welcher mindestens eine frei laufende Messrolle (8, 9) zur Umlenkung des Prüfmaterials (10) um den genannten Winkel (β) angeordnet ist, **dadurch gekennzeichnet, dass** mindestens eine der Messrollen (8, 9) mit einem Inkrementgeber (19 bzw. 20) ausgerüstet ist, durch welchen eine Umwandlung der auf die Messrollen (8, 9) als Drehbewegung übertragene Dehnung des Prüfmaterials in den Bereichen (10', 10") zwischen Klemmvorrichtung (2, 9) und benachbarter Messrolle (8, 9) einschliesslich des aus den Klemmvorrichtungen (2, 4) herauskriechenden Klemmenschlupfes (Δ1) in dehnungsproportionale Signale erfolgt, dass weiters ein die Veränderung des Abstandes der Klemmvorrichtungen (2, 4) überwachender Inkrementgeber (17) vorgesehen ist, und dass die von diesem erfassten Dehnungswerte in dehnungsproportionale Signale umgewandelt und zusammen mit den dehnungsproportionalen Signalen des zumindest einen genannten Inkrementgebers (19, 20) einem Auswertegerät (34) zugeführt sind..

4. Vorrichtung nach Anspruch 3, **dadurch gekennzeichnet, dass** zwei Messrollen (8, 9) vorgesehen sind, von denen mindestens eine mit einer Kraftmesszelle (3) in Verbindung steht.

5. Vorrichtung nach Anspruch 4, **dadurch gekennzeichnet, dass** mindesten eine Messrolle (8, 9) mit der Kraftmesszelle (3) über eine Lasche (6) in Verbindung steht.

6. Vorrichtung nach Anspruch 5, **dadurch gekennzeichnet, dass** die Lasche (6) in der Vektorrichtung der resultierenden Kraft (R) wirkt, und dass über die Lasche (6) eine Übertragung der resultierenden Kraft (R) auf die Krafteinleitungsfläche (7) der Kraftmesszelle (3) erfolgt.

7. Vorrichtung nach einem der Ansprüche 3 bis 6, **dadurch gekennzeichnet, dass** die mindestens eine Messrolle (8, 9) eine mit Leitflächen (27', 27", 33) versehene Abdeckung (27) zur Ablenkung der zurückschlagenden Enden nach dem Bruch des Prüfmaterials (10) aufweist.

## Claims

1. A method for determining strength properties of an elongated textile test material (10), in which the test material (10) is clamped and stretched between a stationary and a movable clamping device (2 and 4, respectively) and, between the clamping devices (2, 4), the test material (10) is deflected by at least one free-running measuring roller (8, 9) by an angle (β), **characterised in that** the total extension of the test material (10) including clamp slippage (Δ1) of the test material (10), and the extension of the test material (10) at least in one of the regions between clamping device (2, 4) and adjacent measuring roller (8, 9) including the clamp slippage (Δ1) is converted into signals which are proportional to the extension, and **in that** these signals are evaluated.

2. A method according to Claim 1, **characterised in that** a force couple (P, P') is formed due to the deflection of the test material (10) by an angle (β), the resultant force (R) of the force couple being used for determining the strength properties.

3. A device for carrying out the method according to Claim 1 or 2, with a stationary and a movable clamping device (2 and 4, respectively) between which a measuring section (l₀) is formed, within which at least one free-running measuring roller (8, 9) is disposed for deflecting the test material (10) by the said angle (β), **characterised in that** at least one of the measuring rollers (8, 9) is equipped with an incremental transducer (19 and 20, respectively) by means of which the extension of the test material in the sections (10', 10") between a clamping device (2, 9) and an adjacent measuring roller (8, 9), including the clamp slippage (Δ1) creeping out of the clamping devices (2, 4), which is transferred to the measuring rollers (8, 9) as rotational movement, is converted into signals which are proportional to the extension, **in that** furthermore an incremental transducer (17) is provided which monitors the change in the distance between the clamping devices (2, 4) and **in that** the extension values measured by it are converted into signals which are proportional to the extension and are delivered to an evaluator (34) together with the extension-proportional signals of said at least one incremental transducer (19, 20).

4. A device according to Claim 3, **characterised in that** two measuring rollers (8, 9) are provided, at least one of which is connected to a load cell (3).

5. A device according to Claim 4, **characterised in that** at least one measuring roller (8, 9) is connected to the load cell (3) via a link (6).

6. A device according to Claim 5, **characterised in that** the link (6) acts in a vector direction of the resultant force (R) and **in that** the resultant force (R) is transferred to a force transfer face (7) of the load cell (3) via the link (6).

7. A device according to any one of Claims 3 to 6, **characterised in that** the at least one measuring roller (8, 9) has a cover (27) provided with guide faces (27', 27", 33) for the purpose of deflecting recoiling ends following breakage of the test material (10).

## Revendications

1. Procédé pour la détermination des caractéristiques mécaniques d'une matière d'essai textile allongée (10), dans lequel la matiere d'essai (10) est serrée et étendue entre un moyen de serrage fixe et un moyen de serrage mobile (2 respectivement 4) et la matière d'essai (10) est tournée d'un angle (β) entre lesdits moyens de serrage (2, 4) par au moins un rouleau de mesure (8, 9) tournant librement, **caractérisé en ce que** l'extension totale de la matière d'essai (10), y compris le glissement de serrage (Δ1) de la matière d'essai (10), et l'extension de la matière d'essai (10) au moins dans une des zones entre le moyen de serrage (2, 4) et le rouleau de mesure (8, 9) voisin, y compris le glissement de serrage (Δ1), sont transformées en signaux proportionnels à l'extension, et **en ce que** lesdits signaux sont évalués.

2. Procédé selon la revendication 1, **caractérisé en ce qu'**un moment de force (P, P') est créé par la déflection de la matière d'essai (10) d'un angle (β), dont la force résultante (R) est utilisée à déterminer les caractéristiques mécaniques.

3. Dispositif pour la mise en oeuvre du procédé selon la revendication 1 ou 2, dispositif qui comprend un moyen de serrage fixe et un moyen de serrage mobile (2 respectivement 4), entre lequels est formée une distance mesurée (lₒ) dans laquelle au moins un rouleau de mesure (8, 9) tournant librement est diposé pour tourner la matière d'essai (10) dudit angle (Δ), **caractérisé en ce qu'**au moins un desdits rouleaux de mesure (8, 9) est pourvu d'un transmetteur de mesure incrémentiel (19 respectivement 20) qui sert à transformer en signaux proportionnels à l'extension, l'extension de la matière d'essai transmise auxdits rouleaux de mesure (8, 9) en tant que mouvement rotatif dans les zones (10', 10") entre le moyen de serrage (2, 4) et le rouleau de mesure (8, 9) voisin, y compris le glissement de serrage (Δ1) sortant des moyens de serrage (2, 4), **en ce qu'**un transmetteur de mesure incrémentiel (17) est, de plus, prévu pour surveiller le changement de la distance desdits moyens de serrage (2, 4) et **en ce que** les valeurs d'extension detectées par celui-ci sont transformées en signaux proportionnels à l'extension et sont amenées à un appareil d'évaluation (34) conjointement avec les signaux proportionnels à l'extension dudit au moins un transmetteur de mesure incrémentiel (19, 20).

4. Dispositif selon la revendication 3, **caractérisé en ce que** deux rouleaux de mesure (8, 9) sont prèvus, dont au moins un est en communication avec un capteur de force (3).

5. Dispositif selon la revendication 4, **caractérisé en ce qu'**au moins un desdits rouleaux de mesure (8, 9) est en communication avec ledit capteur de force (3) par l'intermédiaire d'une éclisse (6).

6. Dispositif selon la revendication 5, **caractérisé en ce que** l'éclisse (6) agit dans la direction vectorielle de la force résultante (R) et **en ce qu'**une transmission de la force résultante (R) à la surface d'introduction de force (7) du capteur de force (3) est effectuée par l'intermédiaire de l'éclisse (6).

7. Dispositif selon l'une des revendications 3 à 6, **caractérisé en ce que** ledit au moins un rouleau de mesure (8, 9) est pourvu d'une couverture (27) comportant des surfaces de guidage (27', 27", 33) qui servent à la déviation des extrémités retournant après la fracture de la matière d'essai (10).
